# FASCICULE DE BREVET EUROPEEN

(11) **EP 0 642 531 B1**
(45) Date de publication et mention de la délivrance du brevet: **26.03.1997**
(21) Numéro de dépôt: 93910135.8
(22) Date de dépôt: 18.05.1993
(51) Int. Cl.: C07K 7/06, C07K 7/08, C07K 14/00, A61K 38/04, A61K 38/16

(54) **PEPTIDES MONOMERES ET DIMERES, UTILISATION EN TANT QU'AGENTS CYTOPROTECTEURS ET PROCEDE DE PREPARATION**
Monomere und dimere Peptide, ihre Verwendung zum Schutz von Zellen und ihre Herstellungsverfahren
MONOMER AND DIMER PEPTIDES, UTILIZATION AS CYTOPROTECTOR AGENTS AND PREPARATION METHOD

(30) Priorité: 18.05.1992 FR 9205990
(43) Date de publication de la demande: 15.03.1995
(73) Titulaire: INSTITUT DES VAISSEAUX ET DU SANG, F-75010 Paris (FR)
(72) Inventeur: HAN, Zhong, Chao, F-75010 Paris (FR); CAEN, Jacques, Philippe, F-75007 Paris (FR); AMIRAL, Jean, F-95130 Franconville (FR)
(74) Mandataire: Clisci, Serge
(86) Numéro de dépôt international: FR9300483
(87) Numéro de publication internationale: WO9323426

(56) Documents cités:
- WO-A-91/04274
- WO-A-92/02240
- US-A- 5 185 323
- JOURNAL OF CLINICAL INVESTIGATION vol. 83, no. 5, Mai 1989, pages 1477 - 1486
- A.M. GEWIRTZ ET AL. 'Inhibition of Human Megakaryocytopoiesis in Vitro by Platelet Factor 4 (PF4) and a Synthetic COOH-Terminal PF4 Peptide' cité dans la demande

## Description

### DOMAINE DE L'INVENTION

La présente invention a trait à une nouvelle utilisation en thérapeutique de substances peptidiques comprenant les fragments F₁ et F₂ ci-après en tant qu'agents cytoprotecteurs.

Elle concerne en tant que produits industriels nouveaux les composés de formule I (peptides monomères) et de formule II (peptides dimères) ci-après.

Elle concerne également un procédé de préparation de ces nouveaux peptides monomères et dimères.

### ARRIERE PLAN DE L'INVENTION

On sait que la mégacaryocytopoïèse concerne l'engagement des cellules-souches hématopoïétiques vers la lignée mégacaryocytaire, la prolifération des progéniteurs mégacaryocytaires et la maturation des mégacaryocytes pour donner naissance aux plaquettes ; voir à cet effet les articles de E.M. MAZUR, *Exp. Hematol.,* (1987), 15, 340-350, de R. HOFFMAN, *Blood,* (1989) 74, 1106-1212, et de Z.C. HAN et al., *Int. J. Hematol.,* (1991), 54, 3- 14.

On sait par ailleurs que les plaquettes sanguines jouent un rôle très important dans certains processus physiologiques, notamment dans les mécanismes de l'hémostase ; voir à cet effet l'ouvrage de J.P. CAEN et al., intitulé *"Platelet Physiology and Pathology",* Stratton International, New York, 1977.

La moëlle hématopoïétique est un système complexe formé de cellules de nature et de fonction différentes. Son organisation peut se concevoir en cinq compartiments cellulaires : les cellules-souches de toute la lignée hématopoïétique, les cellules progénitrices hématopoïétiques, les cellules hématopoïétiques matures, le micro-environnement et les cellules accessoires. Les cellules-souches par une série de différenciations et de maturations donnent naissance aux cellules progénitrices dont font partie les progéniteurs mégacaryocytaires, et ces derniers donnent naissance à leur tour aux plaquettes comme indiqué ci-dessus.

La thrombocytémie est un état pathologique dont la multiplication excessive atteint la lignée mégacaryocyto-plaquettaire. Elle est présente dans plusieurs maladies, à savoir en particulier la thrombocytémie essentielle, la polyglobulie, la leucémie myéloïde, la splénomégalie myéloïde et la leucémie mégacaryocytaire. La persistance de la thrombocytémie pose des problèmes de thromboses et d'hémorragies.

### ART ANTERIEUR

Des substances peptidiques comportant dans leur structure des fragments particuliers inclus dans les définitions de F₁ et F₂ données ci-après ont déjà été décrites, à savoir les pF4, C-24, C-41 (deux fragments peptidiques), βTG, CTAP-III et IL8 (la liste des abréviations utilisées est fournie ci-après). Voir notamment à cet effet les publications suivantes :
(a) l'article de A. M. GEWIRTZ et al., intitulé : *"Inhibition of Human Hegakaryocytopoiesis in Vitro by Platelet Factor 4 (pF4) and a Synthetic COOH-terminal pF4 Peptide"* et publié dans *Clin. J. Invest.,* (1989), 83, 1477-1486 ;
(b) l'article de M. B. ZUCKER et al., intitulé *"Immunoregulatory activity of peptides related to platelet factor 4"* et publié dans *Proc. Natl. Acad. Sci. USA,* (1989), 86, 7571-7574 ;
(c) l'article de T. E. MAIONE et al., intitulé : *"Inhibition of Angiogenesis by Recombinant Human Platelet Factor-4 and Related Peptides"* et publié dans *Science,* (1990), 247, 77-79 ; et
(d) les publications EP-A-0 378 364, EP-A-0 407 122 et WO-A-92 02 240 de la société REPLIGEN CORPORATION.

L'article de T. E. MAIONE et al. précité fait état des propriétés anti-angiogénèse du pF4 recombinant humain (rh-pF4) et de ses fragments C-41 et C-13. Il montre que l'activité angiostatique, évaluée par l'inhibition de l'angiogénèse dans la CAM de poulet, est associée à la région COOH-terminale du pF4 qui lie l'héparine, le C-13 se distinguant par le fait qu'il inhibe l'angiogénèse mais ne fixe pas l'héparine ni n'inhibe son activité anticoagulante.

L'article de M. B. ZUCKER et al. précité fait état des propriétés immunorégulatrices du pF4 et de certains de ses fragments ne comportant pas les fragments F₁ et F₂ de la présente invention.

Les demandes de brevet publiées EP-A-0 378 364, EP-A-0 407 122 et WO-A-92 02 240 précitées ont trait à l'utilisation du pF4 et de certains de ses fragments ne comportant pas les fragments F₁ et F₂ de la présente invention, en tant qu'inhibiteurs de l'angiogénèse.

Enfin l'article de A. M. GEWIRTZ et al. précité fait état d'essais *in vitro* avec un pF4 hautement purifié et deux peptides synthétiques correspondant à des fragments du pF4, à savoir :
- le peptide court C-13 constitué par les 13 derniers aminoacides (i.e. 58-70) de l'extrémité C-terminale du pF4, et
- le peptide long C-24 constitué par les 24 derniers aminoacides (i.e. 47-70) de l'extrémité C-terminale du pF4,
en vue d'apprécier leur éventuel effet sur la mégacaryocytopoïèse.

Cet article enseigne que (i) le peptide C-13 n'a aucun effet sur la mégacaryocytopoïèse, (ii) le pF4 hautement purifié et le peptide C-24 inhibent *in vitro* la mégacaryocytopoièse humaine quand ils interviennent à une dose relativement élevée (environ 25 µg/ml), et (iii) les résultats des essais réalisés *in vitro* ne sont pas cependant automatiquement applicables aux mécanismes d'action *in vivo.*

### BUT DE L'INVENTION

Dans le domaine thérapeutique, il existe un premier besoin en ce qui concerne des produits cytoprotecteurs permettant de protéger efficacement l'organisme, notamment ses cellules, quand il est soumis à des agressions médicalement nécessaires (chimiothérapies, autogreffes) ou à des agressions involontaires (exposition à des radicaux libres, des rayonnements ionisants, des rayonnements bêta, des rayonnements gamma).

Par ailleurs, il existe un second besoin, à savoir, d'une part, en moyen inhibiteur de la mégacaryocytopoïèse pour le traitement des thrombocytémies, et d'autre part, en outil biologique pour tester et mesurer la mégacaryocytopoïèse.

On se propose de satisfaire le premier besoin en faisant appel à une nouvelle solution technique mettant en oeuvre, en tant que moyens cytoprotecteurs, des produits déjà connus selon l'art antérieur dans d'autres utilisations et des produits nouveaux, ces produits comportant dans leur structure les fragments F₁ et F₂ définis ci-après.

De façon avantageuse, cette nouvelle solution technique permet également de satisfaire le second besoin en fournissant de nouveaux produits qui sont efficacement à la fois cytoprotecteurs et anti-mégacaryocytaires in vivo.

### OBJET DE L'INVENTION

La nouvelle solution technique que l'on préconise selon l'invention, fait appel à des substances peptidiques particulières.

Suivant un premier aspect de l'invention, on préconise, une nouvelle utilisation d'une substance peptidique, ladite utilisation étant caractérisée en ce que l'on fait appel à une substance peptidique comportant dans sa structure
(a) un premier fragment de formule :

   Asx-A₁-A₂ (F₁)

   dans laquelle,
   Asx représente Asp ou Asn,
   A₁ représente Gly, MeGly, EtGly ou Aib,
   A₂ représente Arg, Dbu, His, Lys ou Orn, et
(b) un second fragment de formule

   A₃-Cys-A₄-Asx (F₂)

   (voir SEQ ID NO : 1) dans laquelle, A₃ représente Leu, Ile, Nle, Val, Nva, Ahe, Acp ou Cle,
A₄ est Leu, Ile, Nle, Val, Nva, Ahe, Acp ou Cle, et Asx représente Asp ou Asn comme indiqué ci-dessus, le second fragment (F₂) étant plus proche de l'extrémité C-terminale que le premier fragment (F₁), pour la préparation d'un médicament cytoprotecteur utile vis-à-vis des agressions cytotoxiques.

Suivant un second aspect de l'invention, l'on préconise en tant que produit industriel nouveau, une substance peptidique qui est caractérisée en ce qu'elle a une structure monomère de formule

R-B₁-Asx-A₁-A₂-A₅-A₃-Cys-A₄-Asx-B₂-R' (I)

(voir SEQ ID NO : 3) ou une structure dimère de formule 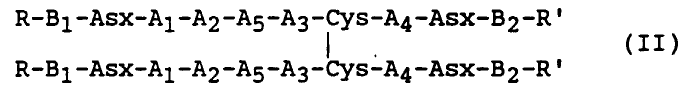 où A₁, A₂, A₃, et A₄ sont définis comme indiqué ci-dessus,
A₅ représente un groupe aminoacide ayant un groupe latéral basique, acide ou amide,
B₁ représente une simple liaison, un groupe aminoacide ou un reste peptidique ayant de 2 à 10 groupes aminoacides,
B₂ représente un groupe aminoacide ou un reste peptidique ayant de 2 à 14 groupes aminoacide, l'aminoacide de l'extrémité C-terminale de B₂ ayant une structure cyclique et étant choisi parmi l'ensemble constitué par Pro, 3Hyp, 4Hyp, ATC, Aze,Pip, Cle, ACC et Pyr.
R est H ou un groupe protecteur de la fonction NH₂ de l'extrémité N-terminale,
R' est OH ou un groupe protecteur de la fonction COOH de l'extrémité C-terminale.

Suivant un troisième aspect de l'invention, l'on préconise un procédé de préparation des peptides de formule I (monomères) et II (dimères).

On a découvert selon l'invention l'existence d'une cytoprotection dans la lignée mégacaryocytaire, et l'on préconise donc également suivant encore un autre aspect une utilisation des peptides monomères de formule I et des peptides dimères de formule II en tant qu'inhibiteurs de la mégacaryocytopoïèse, tant en thérapeutique humaine et vétérinaire (chez les animaux à sang chaud et en particulier les mammifères) que dans le domaine du diagnostic.

### ABREVIATIONS

Par commodité, on a utilisé dans la présente description les abréviations suivantes
(a) en ce qui concerne les aminoacides ou leurs restes :
   - Aad =: acide 2-aminoadipique
   - Abu =: acide 2-aminobutyrique
   - 4Abu =: acide 4-aminobutyrique
   - ACC =: acide 1-aminocyclohexane-1-carboxylique
   - Acp =: acide 6-aminocaproïque
   - Ahe =: acide 2-aminoheptanoique
   - Aib =: acide 2-aminoisobutyrique
   - Ala =: alanine
   - Arg =: arginine
   - Asn =: asparagine
   - Asp =: acide aspartique
   - Asx =: Asn ou Asp
   - ATC =: acide 4-thiazolidinecarboxylique (i.e. acide timonacique ou thioproline)
   - Aze =: azétidine (i.e. plus précisément : acide 2-azétidine-carboxylique)
   - CHA =: cyclohexylalanine [i.e. acide 2-amino-3-(cyclohexylméthyl)propionique]
   - CHG =: cyclohexylglycine [i.e. acide 2-amino-3-(cyclohexyl)propionique]
   - CHT =: (4-hydroxycyclohexyl)alanine [i.e. acide 2-amino-3-(4-hydroxycyclohexylmethyl)-propionique]
   - Cle =: cycloleucine (i.e. acide 1-aminocyclopentane-1-carboxylique)
   - CHU =: (4-hydroxycyclohexyl)glycine [i.e. acide 2-amino-3-(4-hydroxycyclohexyl)-propionique]
   - CPA =: cyclopentylalanine [i.e. acide 2-amino-3-(cyclopentylméthyl)-propionique]
   - CPG =: cyclopentylglycine [i.e. acide 2-amino-3-cyclopentylpropionique]
   - Cys =: cystéine
   - Dbu =: acide 2,4-diaminobutyrique
   - Dpr =: acide 2,3-diaminopropionique
   - EtGly =: N-éthylglycine
   - Gln =: glutamine
   - Glu =: acide glutamique
   - Gly =: glycine
   - Glx =: Gln ou Glu
   - His =: histidine
   - Hyp =: hydroxyproline (3Hyp ou 4Hyp)
   - 3Hyp =: 3-hydroxyproline
   - 4Hyp =: 4-hydroxyproline
   - Ile =: isoleucine
   - Leu =: leucine
   - Lys =: lysine
   - MeGly =: N-méthylglycine (ou sarcosine)
   - Met =: méthionine
   - Nle =: norleucine
   - Nva =: norvaline
   - Orn =: ornithine
   - Phg =: phénylglycine [i.e. acide 2-amino-3-phényl-1-propionique]
   - Phe =: phénylalanine
   - Pip =: acide pipécolique [i.e. acide 2-pipéridinecarboxylique]
   - Pro =: proline
   - Pyr =: acide 2-pyrrolidone-5-carboxylique
   - Ser =: sérine
   - Thr =: thréonine
   - Trp =: tryptophane
   - Tyr =: tyrosine
   - Val =: valine
(b) en ce qui concerne les autres abréviations :
   - Adoc =: adamantyloxycarbonyle
   - Aoc =: t-amyloxycarbonyle
   - BSA =: albumine sérique bovine
   - CAM =: membrane chorioallantoïque (de l'anglais : "chorioallantoic membrane")
   - Cbo =: carbobenzoxy
   - C-13 =: peptide constitué par les 13 derniers aminoacides (i.e. 58-70) de l'extrémité C-terminale du pF4,
   - C-24 =: peptide constitué par les 24 derniers aminoacides (i.e. 47-70) de l'extrémité C-terminale du pF4,
   - C-41 =: peptide constitué par les 41 derniers aminoacides (i.e. 30-70) de l'extrémité C-terminale du pF4,
   - CFU =: unité formant colonie (de l'anglais : "colony-forming unit")
   - CFU-MK =: progéniteur mégacaryocytaire (de l'anglais : "megakaryocyte colony-stimulating unit")
   - CTAP-III =: protéine (apoprotéine) 3 du tissu conjonctif (la séquence des aminoacides de CTAP-III est similaire de celle de βTG avec la seule différence qu'elle contient 4 aminoacides supplémentaires (Asn-Leu-Ala-Lys) à l'extrémité N-terminale)
   - Fmoc =: 9-fluorenylméthyloxycarbonyle
   - Foc =: furfuryloxycarbonyle
   - 5FU =: 5-fluorouracile, produit cytotoxique de référence
   - HPP-CFC =: cellule formant des colonies à haut potentiel prolifératif (en anglais : "high proliferative potential colony-forming cell")
   - HEL =: érythroleucémie humaine
   - HPLC =: chromatographie liquide de haute performance
   - Iboc =: isobornyloxycarbonyle
   - Il8 =: interleukine 8
   - LA-pF4 =: précurseur de βTG (de anglais : "low-affinity pF4")
   - 2ME =: 2-mercaptoéthanol
   - MEM =: milieu essentiel minimal
   - αMEM =: MEM modifié
   - MK =: mégacaryocyte
   - PB =: plasma bovin citraté
   - PBS =: tampon phosphate salin
   - pF4 =: facteur plaquettaire 4 (de l'anglais : "platelet factor 4")
   - PMA =: myristate/acétate de phorbol
   - RT =: température ambiante (15-20°C)
   - SDS-PAGE =: électrophorèse sur gel de dodécylsulfate de sodium-polyacrylamide (de l'anglais : "sodium dodecylsulphate-polyacrylamide gel electrophophoresis")
   - SP =: sérum aplasique de porc
   - βTG =: bêta-thromboglobuline (produit de dégradation partielle de LA-pF4)
   - TGFβ =: facteur béta de croissance transformant (de l'anglais : "transforming growth factor β")
   - Tos =: p-toluènesulfonyle (ou tosyle)
   - vWF =: facteur von WILLEBRAND
   - Z =: benzyloxycarbonyle
   - Z(p-Cl) =: p-chlorobenzyloxycarbonyle
   - Z(p-OMe) =: p-méthoxybenzyloxycarbonyle

### DESCRIPTION DETAILLEE DE L'INVENTION

Les substances peptidiques selon l'invention, qui sont utiles en tant qu'agents cytoprotecteurs, peuvent présenter dans leur structure un couple F₁/F₂ ou plusieurs couples F₁/F₂. En particulier, la présence de Cys dans F₂ peut conduire à une dimérisation par formation d'un pont S-S entre les Cys de deux chaînes monomères.

Parmi lesdites substances peptidiques qui comportent un couple F₁/F₂, on peut notamment mentionner le pF4 natif, le pF4 recombinant, le fragment C-41 du pF4, βTG, CTAP-III et IL8, qui sont des produits déjà connus en soi, d'une part, et les composés de formules I et II, qui sont des produits nouveaux, d'autre part.

En pratique, les substances peptidiques utiles en tant qu'agents cytoprotecteurs, que l'on préfère suivant l'invention, sont des produits antimégacaryocytaires.

De façon également pratique, les fragments F₁ et F₂ selon l'invention sont liés entre eux par un aminoacide. En d'autres termes, les substances peptidiques selon l'invention, sont avantageusement telles que le fragment F₁ soit relié au fragment F₂ par l'intermédiaire d'un amino acide A₅ suivant la structure (F₃).

F₁-A₅-F₂ (F₃)

(voir SEQ ID No : 2) dans laquelle A₅ est un reste aminoacide comportant un groupe latéral amino NH₂, acide COOH ou amide CONH₂, et F₁ et F₂ sont définis comme indiqué ci-dessus.

De façon avantageuse, A₅ représentera Arg, Lys, His, Orn, Dbu, Glu, Gln, Asp, Asn, Aad ou Dpr.

Comme indiqué ci-dessus, les composés monomères de formule I et les composés dimères de formule II, qui en dérivent, comportent (i) un aminoacide A₅ situé entre les fragments F₁ et F₂, (ii) un fragment B₂ situé au niveau de l'extrémité C-terminale et ayant de 1 à 4 groupes aminoacides, et (iii) un groupe B₁ du côté de l'extrémité N-terminale représentant une simple liaison, un groupe monoaminoacide ou un groupe peptide de 2 à 10 groupes aminoacides.

Quand B₁ représente un reste aminoacide ou un reste peptide, le ou les aminoacides qui le composent sont quelconques à l'exclusion du groupe Cys (qui est susceptible de donner lieu à des dimérisations).

L'aminoacide de B₂ situé à l'extrémité C-terminale du peptide I ou II sera avantageusement un aminoacide cyclique et notamment choisi parmi l'ensemble constitué par Pro, 3Hyp, 4Hyp, ATC, Aze, Pip, Cle, ACC et Pyr.

Parmi les aminoacides inclus dans B₁, d'une part, et dans B₂ à l'exclusion de l'extrémité C-terminale, d'autre part, on peut notamment citer ceux qui sont choisis parmi l'ensemble constitué par Aad, Abu, 4Abu, ACC, Acp, Ahe, Aib, Ala, Arg, Asn, Asp, ATC, Aze, CHA, CHG, CHT, Cle, CHU, CPA, CPG, Dbu, Dpr, EtGly, Gln, Glu, Gly, His, 3Hyp, 4Hyp, Ile, Leu, Lys, MeGly, Met, Nle, Nva, Orn, Phg, Phe, Pip, Pro, Pyr, Ser, Thr, Trp, Tyr et Val ; on préfère plus particulièrement suivant l'invention les aminoacides choisis parmi Ala, Phe, CHA, CHG, CPA, CPG, Tyr, CHU, CHT, Leu, Ile, Nle, Val, Nva, Ahe, Acp, Arg, Lys, Orn, Dbu, Glu, Gln, Asp, Asn, Aad, Dpr, Ser, Gly, MeGly, EtGly, Aib, Thr, Pip, Pro, 3Hyp, 4Hyp, ATC, Aze, Cle, ACC et Pyr.

A l'exception de quelques groupes d'aminoacides (notamment Gly, MeGly, EtGly, Acp, 4Abu et Aib) tous les autres alpha-aminoacides des molécules du composé monomère de formule I et du composé dimère de formule II comportent un atome de carbone asymétrique, et sont chacun de configuration L ou D. D'un point de vue pratique, l'on préfère selon l'invention que (i) tous les alpha-aminoacides à atome de carbone asymétrique présents dans les structures de formule I et de formule II mais non situés sur l'extrémité N-terminale soient de configuration L, et (ii) l'alpha-aminoacide de l'extrémité N-terminale soit de configuration D ou L.

Le groupe R représente soit l'atome d'hydrogène de l'extrémité N-terminale, soit un groupe protecteur de ladite extrémité N-terminale bien connu dans le domaine des peptides. Un tel groupe protecteur peut intervenir dans la synthèse des composés de formules I et II ou dans l'amélioration des propriétés desdits composés. Parmi les groupes protecteurs qui conviennent, on peut notamment citer les groupes Adoc, Aoc, Cbo, Fmoc, Foc, Iboc, Tos, Z, Z(p-Cl), Z(p-OMe), méthyloxymalonyle, éthyloxymalonyle, phényloxymalonyle, 4-chlorophényloxymalonyle, benzyloxymalonyle. On préfère plutôt selon l'invention que les composés de formules I et II n'aient pas leurs extrémités N-terminales protégées.

Le groupe R' représente soit OH pour compléter le groupe carboxylique de l'extrémité C-terminale, soit un groupe protecteur de ladite extrémité C-terminale bien connu dans le domaine des peptides, par exemple un groupe ester. Le groupe protecteur R' intervient principalement dans la synthèse peptidique, il n'est pas particulièrement essentiel ni critique dans les activités cytoprotectrices et antimégacaryocytaires. De préférence, les peptides de formules I et II ne comporteront pas de groupe protecteur sur l'extrémité C-terminale.

Dans la structure des composés de formules I et II ainsi que dans celles des fragments F₁, F₂ et F₃, on peut eu égard aux définitions données ci-dessus rencontrer des aminoacides présentant un groupe latéral basique, hydroxyle ou acide. Sans sortir du cadre de l'invention, il est clair que chacun de ces groupes latéraux peut être protégé. Ainsi un groupe latéral basique peut être protégé notamment par Cbo, un groupe latéral hydroxyle peut être protégé sous la forme éther ou ester, et un groupe latéral acide peut être protégé sous la forme ester.

La dimérisation de I en II par formation d'un pont disulfure entre les Cys de 2 chaînes monomères, s'initie spontanément en milieu aqueux à RT. Pour éviter cette dimérisation en milieu aqueux, on a intérêt à introduire dans ledit milieu aqueux un moyen antioxydant, un moyen bloquant la fonction latérale -SH de Cys ou un moyen clivant le pont disulfure selon le mécanisme :

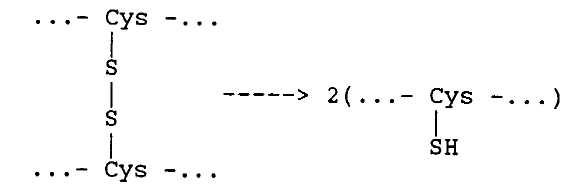

Les composés peptides de formule I à l'état sec, notamment obtenus par lyophilisation après purification, sont relativement moins sensibles à ladite dimérisation que les mêmes composés en milieu aqueux, voire même substantiellement insensibles à ladite dimérisation.

Selon un mode avantageux pour la préparation d'un peptide monomère de formule I, l'on fait appel à un procédé, qui met en oeuvre une synthèse peptidique séquentielle dans un milieu réactionnel hétérogène et une étape de purification, comprenant l'utilisation d'un agent réducteur
(i) dans le milieu réactionnel à partir de l'instant où Cys intervient dans la synthèse dudit peptide monomère, ou ultérieurement
(ii) avant et/ou après purification,
afin de réduire substantiellement ou d'empêcher la production du peptide dimère de formule II qui résulte de la formation d'un pont disulfure -S-S- entre les Cys de deux peptides monomères.

De façon préférée, ledit agent réducteur sera introduit dans le milieu réactionnel à partir de l'instant où Cys intervient dans la synthèse du peptide monomère, d'une part, et sera présent dans ledit milieu au moins jusqu'à l'étape de purification, d'autre part.

D'un point de vue pratique, ledit agent réducteur sera choisi parmi l'ensemble constitué par les substances clivant le pont disulfure -S-S-, les substances protégeant l'extrémité -SH du groupe latéral de Cys et leurs mélanges.

De préférence, ledit agent réducteur sera choisi parmi l'ensemble constitué par
(1) le dithiothréitol qui est utilisé à partir de l'instant où Cys intervient dans la synthèse, avant et/ou après purification, et
(2) les composés iodoacétiques qui sont utilisés après purification, notamment l'acide iodoacétique ou l'iodoacétamide.

Selon un mode particulier, que l'on recommande pour la préparation d'un composé monomère de formule I, le procédé que l'on préconise comprend en outre l'utilisation, avant et/ou après purification, d'un agent antioxydant, notamment un composé ascorbique (notamment l'acide ascorbique, ou un ascorbate de métal alcalin, de métal alcalino-terreux ou d'ammonium) et de préférence un tampon acide ascorbique/ascorbate de sodium ou acide ascorbique/ ascorbate de potassium.

Pour la préparation des composés dimères de formule II, il suffit de réaliser la même synthèse peptidique sans se préoccuper d'incorporer un agent réducteur dans le milieu réactionnel lors de l'introduction du groupe Cys.

En variante, quand on veut synthétiser des composés dimères de formule II ayant des séquences très similaires, il est recommandé de préparer (i) le fragment commun desdites séquences, puis de laisser la dimérisation s'effectuer, ou (ii) les composés monomères correspondants selon le procédé décrit ci-dessus, puis de laisser la dimérisation s'effectuer.

Le matériau peptidique selon l'invention, qu'il soit sous forme monomère de formule I, sous forme dimère de formule II ou sous la forme d'un mélange monomère/dimère, est utile notamment *in vivo* en tant que substance cytoprotectrice, d'une part, et substance anti-mégacaryocytaire, d'autre part.

On préconise donc en premier lieu, une utilisation qui est caractérisée en ce que l'on fait appel à une substance peptidique comprenant les fragments F₁ et F₂ selon l'invention, en tant que substance cytoprotectrice vis-à-vis des agressions cytotoxiques, notamment celles induites par les chimiothérapies et les autogreffes. On a en effet pu observer que les cellules des animaux à sang chaud, notamment chez l'homme et les mammifères, sont protégées de façon efficace par les substances peptidiques selon l'invention, en ce sens qu'après la fin de l'agression les cellules des sujets traités avec une substance peptidique selon l'invention redeviennent à leur niveau normal plus rapidement que celles des sujets ne recevant pas ladite substance peptidique.

On préconise en second lieu, une autre utilisation qui est caractérisée en ce que l'on fait appel à un composé choisi parmi les peptides monomères de formule I, les peptides dimères de formule II et leurs mélanges, en tant que substance anti-mégacaryocytaire pour l'obtention d'un médicament anti-thrombocytémie ou d'un outil pour le diagnostic des thrombocytémies.

On préconise en troisième lieu une utilisation qui est caractérisée en ce que l'on fait appel à un composé choisi parmi les peptides monomères de formule I, les peptides dimères de formule II et leurs mélanges, en tant que substance anti-mégacaryocytaire dans des tests biologiques concernant la détermination de la mégacaryocytopoièse.

En thérapeutique humaine et vétérinaire (c'est-à-dire chez l'homme et les animaux à sang chaud, notamment les animaux mammifères), les composés de formule I et II interviennent *in vivo* en tant que régulateurs négatifs (i.e. inhibiteurs) de la mégacaryocytopoièse, et se sont révélés particulièrement efficaces vis-à-vis du traitement des thrombocytémies, notamment les composés des exemples 1 (monomère de formule Ia) et 2 (dimère de formule IIa).

Les thrombocytémies comprennent en particulier
- la thrombocytémie dite essentielle,
- la thrombocytémie d'autres types de syndromes myéloprolifératifs (notamment la polyglobulie, la leucémie myéloïde chronique et la splénomégalie myéloïde),
- la leucémie mégacaryocytaire, et
- les thrombocytoses observées dans un certain nombre de cancers et d'autres états pathologiques.

En thérapeutique, la posologie quotidienne que l'on préconise pour chacune des substances peptidiques selon l'invention est de 1 µg/kg à 10 mg/kg.

En tant qu'agents cytoprotecteurs, les substances peptidiques comportant les fragments F₁ et F₂ selon l'invention, notamment les peptides monomères de formule I, les peptides dimères de formule II et leurs mélanges, conviennent chacune pour administration par voie injectable (en particulier par voie intraveineuse, intramusculaire ou sous-cutanée) suivant une posologie quotidienne de 1 µg/kg à 0,5 mg/kg, et par voie orale suivant une posologie quotidienne de 2 µg/kg à 1 mg/kg.

En tant qu'agents antimégacaryocytaires, les composés de formule I, de formule II ou leurs mélanges, conviennent chacun par voie injectable suivant une posologie quotidienne de 40 µg/kg à 1 mg/kg, et par voie orale suivant une posologie quotidienne de 40 µg/kg à 10 mg/kg (sous forme de comprimés, gélules, nanoparticules, le cas échéant en association avec des liposomes), dans le traitement desdites thrombocytémies ; de façon pratique, on utilisera dans ce cas par voie injectable une dose unitaire quotidienne de 40 µg/kg à 1 mg/kg, et par voie orale, sous une forme gastrorésistante, une dose unitaire quotidienne de 40 µg/kg à 10 mg/kg.

On a consigné ci-après un certain nombre de composés typiques de formule I et de formule II selon l'invention.

### MEILLEUR MODE

Le meilleur mode de mise en oeuvre de l'invention consiste à faire appel à un matériau peptidique qui est constitué par le composé dodécapeptide de formule Ia, (Ex 1), son dimère de formule IIa (Ex 2) et leurs mélanges. Le monomère et le dimère ont ici pratiquement des mêmes activités similaires dans l'organisme pour une même dose donnée, l'ingrédient qui est actif dans l'organisme étant, dans l'état actuel des connaissances, le peptide dimère.

Les produits des exemples 1 et 2 sont particulièrement utiles en tant qu'agents antimégacaryocytaires, d'une part, et agents cytoprotecteurs, d'autre part.

Il convient en outre de remarquer que le dodécapeptide de formule Ia (Ex 1) selon l'invention et son dimère de formule IIa (Ex 2) se différencient de l'enseignement de l'article précité de A.M. GEWIRTZ et al., par le fait que les composés selon l'invention, utilisés *in vitro,* inhibent la mégacaryocytopoïèse à des doses beaucoup plus faibles (notamment à 5 µg/ml voire à 0,1 µg/ml), l'inhibition de la mégacaryocytopoïèse se manifestant par un effet anti-prolifération des cellules HEL sans action sur la maturation desdites cellules HEL, alors que le peptide C-24 et le pF4 hautement purifié limitent la maturation desdites cellules HEL mais n'agissent pas sur leur prolifération. Les produits des exemples Ex 1 et Ex 2 exercent des propriétés anti-mégacaryocytopoïèse *in vivo,* ces propriétés étant différentes des propriétés anti-angiogénèse observées dans l'art antérieur.

D'autres avantages et caractéristiques de l'invention seront mieux compris à la lecture qui va suivre d'exemples de préparation, d'une part, et d'essais pharmacologiques et biologiques, d'autre part. Bien entendu, l'ensemble de ces éléments n'est nullement limitatif mais est fourni à titre d'illustration.

### EXEMPLE 1

Préparation du dodécapeptide de formule Ia où les aminoacides en position 1 et 3-12 sont de configuration L.

Ce produit est préparé selon la méthode classique de synthèse peptidique en phase hétérogène avec fixation sur un support solide. Au moment où l'aminoacide Cys va intervenir dans la synthèse, on introduit dans le milieu réactionnel une substance réductrice, dans le cas d'espèce, le dithiothréitol. Cette substance réductrice est présente dans le milieu réactionnel jusqu'à ce que le dernier aminoacide ait été fixé sur la chaîne peptidique. On ajoute alors un antioxydant, le tampon acide ascorbique/ascorbate de potassium. On procède à la séparation du peptide formé de son support en présence dudit tampon. Le peptide ainsi séparé de son support est purifié par HPLC ou SDS-PAGE en présence dudit tampon acide ascorbique/ascorbate de potassium ; il est ensuite obtenu à l'état sec par lyophilisation.

Le matériau peptidique ainsi obtenu renferme plus de 99 % en poids du peptide monomère désiré.

### EXEMPLE 1 bis

Préparation d'un dodécapeptide de formule Ia où l'aminoacide en position 1 est de configuration D et les aminoacides en positions 3-12 sont de configuration L.

On procède comme indiqué à l'exemple 1 en remplaçant L-Asn par D-Asn et en incorporant dans le produit purifié de l'acide iodoacétique avant lyophilisation.

Le matériau peptidique ainsi obtenu renferme plus de 99 % en poids du peptide monomère désiré.

### EXEMPLE 2

Préparation du composé dimère de formule IIa où les aminoacides en position 1 et 3-12 sont de configuration L.

On procède, comme indiqué l'exemple 1 avec la différence que l'on procède au clivage du peptide de son support en l'absence du tampon antioxydant acide ascorbique/ascorbate de potassium puis effectue la purification par HPLC ou SDS-PAGE toujours en l'absence dudit tampon.

Le matériau peptidique ainsi obtenu renferme plus de 85 % en poids du peptide dimère désiré.

### PARTIE EXPERIMENTALE - MEGACARYOCYTOPOIESE -

### (a) Culture de mégacaryocytes et de leurs progéniteurs

L'effet du matériau peptidique selon l'invention sur la mégacaryocytopoièse *in vitro* a été examiné en utilisant un système de culture de progéniteurs mégacaryocytaires [selon les modalités décrites par HAN et al. dans les articles *Exp. Hematol.,* (1989), 17, 46-52, et *C.R. Acad. Sci. Paris,* (1991), 313, Série III, 553-558]. Dans ce système de culture, les cellules mononucléées murines de moelle de fémur sont prélevées chez les souris Balb/c (IFFA CREDO) et les cellules mononucléées médullaires humaines sont prélevées chez des individus normaux. 2 x 10⁵ cellules médullaires sont ajoutées, au moins en triple, dans un volume de 1 ml d'un milieu de base contenant 10 % (v/v) de plasma bovin citraté, 1 % (v/v) d'albumine bovine, 10 % de 2-meoecaptoéthanol (concentration finale de 10⁻⁴M) et 0,34 mg/ml de CaCl₂ avec ou sans 10 % (v/v) de sérum d'aplasie. Le sérum d'aplasie de porc est utilisé pour les cultures de moelle murine et le sérum d'aplasie humaine est utilisé pour des cultures de moelle humaine. Les colonies mégacaryocytaires murines sont identifiées par la coloration d'acétylcholinestérase [selon la méthode de C.W. JACKSON, *Blood,* (1973), 42, 413-421]. Les colonies mégacaryocytaires humaines sont identifiées par la coloration d'immunoperoxydase en utilisant un anti-corps monoclonal dirigé contre la glycoprotéine IIb/IIIa plaquettaire humaine [voir HAN et al., *Exp. Hematol.,* (1989), 17, 46-52 précité].

### (b) Culture des cellules HEL

Les cellules HEL sont des cellules leucémiques qui expriment différents phénotypes mégacaryocytaires. Elles ont déjà été utilisées comme modèle pour étudier la régulation de la mégacaryocytopoïèse [voir LONG et al., *J. Clin. Invest.,* (1990), 85, 186-195]. La formation des colonies de cellules HEL est réalisée selon la méthode décrite par PIAO et al., *Biochem. Biophys. Res. Commun.,* (1990), 167, 27-32, avec quelques modifications. 4 x 10³ cellules sont cultivées dans un volume de 1 ml contenant 10 % (v/v) de sérum de veau foetal, 10 % (v/v) de PMA 10⁻⁷M, 10 % (v/v) de plasma bovin, 0,34 mg/ml de CaCl₂ et complété par du milieu αMEM (commercialisé par la société EUROBIO). Les cellules sont incubées en atmosphère humide à 37°C avec 5 % de CO₂ pendant 4 jours, puis elles sont comptées au microscope inverse, une colonie HEL contenant un nombre de cellules supérieur à 20.

### (c) Protocole

L'effet du dodécapeptide de l'exemple 1 sur la mégacaryocytopoïèse *in vivo* a été testé sur des souris normales selon une méthode décrite par HAN et al., dans l'article *C.R. Acad. Sci. Paris,* (1991), 313, Série III, 553-558 précité. Chaque groupe contient 5-10 souris Balb/c. Le peptide est injecté par voie sous-cutanée deux fois par jour pendant 4 jours aux concentrations de 1 µg et 5 µg par souris pour chaque administration. Les échantillons de moëlle et/ou de sang ont été prélevés au jour J2 qui suit la dernière injection.

Les souris ont été tuées par dislocation cervicale. Les cellules médullaires ont été obtenues à partir des fémurs et le sang a été obtenu par ponction cardiaque. Les cellules médullaires à la concentration de 2 x 10⁵ cellules mononucléées par ml ont été remises en culture en cinq exemplaires en présence de sérum de porc aplasique pendant une semaine pour tester le nombre de progéniteurs mégacaryocytaires (CFU-MK). Le nombre des mégacaryocytes a été évalué sur des cellules médullaires non cultivées mais ensemencées dans des boîtes de culture après obtention à partir des fémurs. Le nombre de plaquettes, de globules blancs et le taux d'hémoglobine ont été mesurés en utilisant un compteur hématopoiétique automatique.

### (d) Résultats

### Expérimentation in vitro

L'effet du dodécapeptide de l'exemple 1 sur la mégacaryocytopoièse humaine a été testé sur deux types de cellules : les cellules médullaires normales et les cellules HEL.
- Sur les cellules médullaires normales (voir figure 1A), les doses testées ont été de 0,1 µg/ml, 1 µg/ml, 5 µg/ml et 10 µg/ml. La méthode utilisée est celle du caillot plasmatique. Les résultats rapportés représentent la moyenne ± SEM du nombre de colonies MK à partir de 2 expériences.

On constate que le dodécapeptide de l'exemple 1 inhibe la formation des colonies mégacaryocytaires dans les cultures de moëlle normale en présence de sérum aplasique humain.

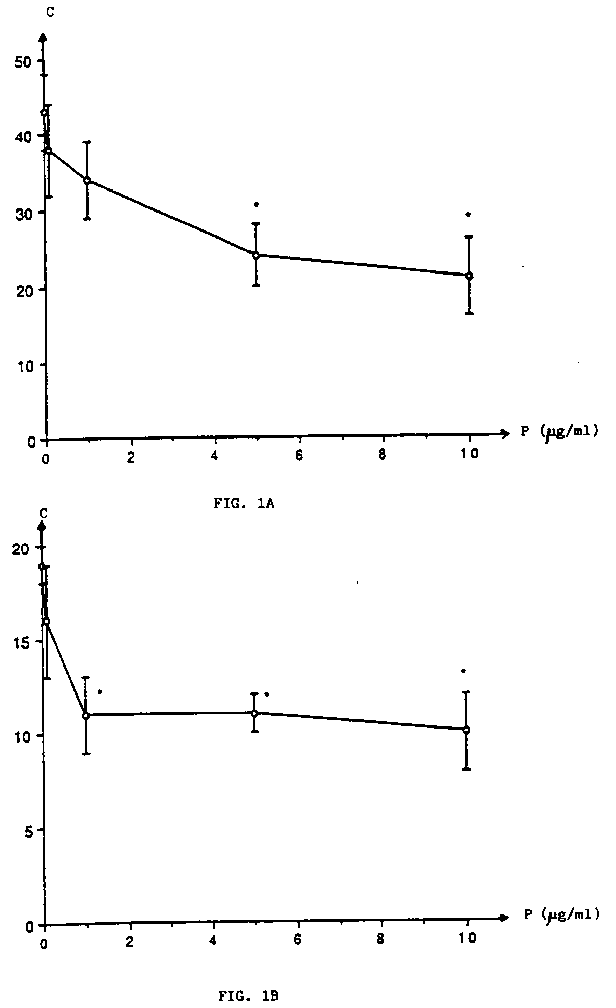

Dans la figure 1A, on a représenté la variation de colonies MK/2 x 10⁵ cellules en ordonnées (C) en fonction de la teneur en peptide (P) exprimée en µg/ml, en abscisses. L'astérisque * indique que le résultat tel qu'observé est statistiquement significatif (p < 0,05 ; test de Student).
- Sur les cellules HEL (voir figure 1B), les doses du dodécapeptide de l'exemple 1 qui ont été testées varient de 0 à 10 µg/ml. Les résultats sont calculés à partir de 3 expériences.

On constate que ledit dodécapeptide de l'exemple 1 inhibe de façon significative la formation des colonies HEL dans ce test.

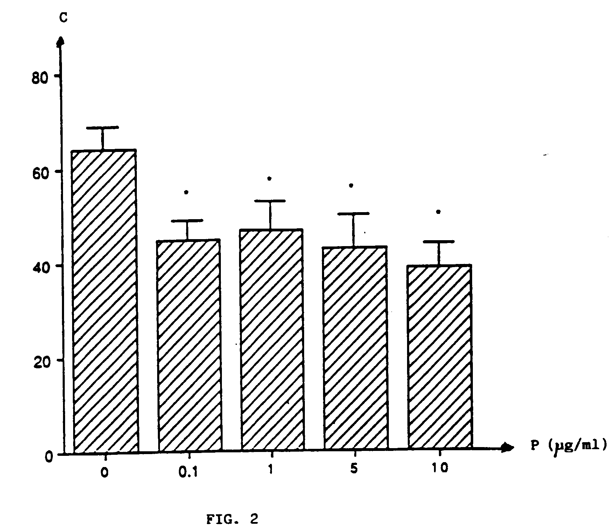

Dans la figure 1B, on a représenté la variation de colonies HEL/10³ cellules en ordonnées (C) en fonction de la teneur en peptide (P) exprimée en µg/ml, en abscisses. L'astérisque * indique que le résultat tel qu'observé est statistiquement significatif (p < 0,05 ; test de Student).
- L'effet du dodécapeptide de l'exemple 1 sur la mégacaryocytopoièse murine a été examiné à des doses variant de 0,1 à 10 µg/ml. Les résultats (voir figure 2) représentent la moyenne ± SEM du nombre de colonies pour 2 x 10⁵ cellules à partir de 3 expériences séparées faites en triple.

Dans la figure 2, on a représenté la variation de colonies MK murins/2 x 10⁵ cellules en ordonnées (C) en fonction de la teneur en peptide (P) exprimée en µg/ml, en abscisses. L'astérisque * indique que le résultat tel qu'observé est statistiquement significatif (p < 0,05 test de Student).

### Expérimentation in vivo

Le dodécapeptide de l'exemple 1 a été injecté par voie sous-cutanée à des souris Balb/c deux fois par jour pendant 4 jours à des concentrations de 0, 40 et 200 µg/kg. Les échantillons de moelle et de sang ont été prélevés le deuxième jour suivant la dernière injection. Les résultats obtenus, qui sont consignés dans le tableau I ci-après, représentent la moyenne ± SEM de trois expériences, chaque groupe de souris contenant au moins 5 animaux.

**TABLEAU I**

| Dose | Moëlle | | Sang | | |
|---|---|---|---|---|---|
| | CFU-MK | MK | L | H | P |
| (µg/kg) | (2 x 10⁵ cellules) | | (10³/µl) | (g/100 ml) | (10³/µl) |
| 0 | 53±5 | 308±18 | 2,67±0,6 | 15,0±0,7 | 937±53 |
| 40 | 41±4* | 289±12 | 2,80±0,4 | 15,2±0,8 | 946±49 |
| 200 | 38±3* | 233±13* | 2,30±0,5 | 14,8±0,9 | 785±53* |

| | | | | | |
|---|---|---|---|---|---|
| Notes L : leucocytes | | | | | |
| H : hémoglobine | | | | | |
| P : plaquettes | | | | | |
| *: statistiquement significatif (p < 0,05 ; test de Student). | | | | | |

On constate que le nombre de CFU-MK médullaires, de mégacaryocytes (MK) isolés et de plaquettes diminue de façon significative deux jours après la dernière injection du dodécapeptide de l'exemple 1 à la dose de 200 µg/kg. On constate également que le nombre de leucocytes et le taux d'hémoglobine ne sont pas affectés.

Les résultats des essais analogues entrepris avec les matériaux peptidiques des exemples 1bis et 2 sont similaires tant *in vitro* que *in vivo,* le produit de l'exemple 2 se montrant cependant plus actif que celui de l'exemple 1.

On a également observé que, sur les cellules HEL, l'inhibition de la mégacaryocytopoièse par les peptides des exemples 1bis et 2 selon l'invention se traduit essentiellement par l'inhibition de la prolifération des cellules et non par l'inhibition de la maturation desdites cellules, à la différence du pF4 hautement purifié et du C-24 de l'article de A.M. GEWIRTZ et al. précité.

### PARTIE EXPERIMENTALE - CYTOPROTECTION -

L'étude de la cytoprotection a été réalisée *in vivo* avec le matériel suivant :
- des souris Balb/c mâles, âgées de 8 semaines sont élevées dans des conditions standards : elles reçoivent chacune de la nourriture pour rongeurs (5 g) et de l'eau (5 ml) par jour ;
- des réactifs pour la culture cellulaire, à savoir : du milieu de EAGLE avec la modification alpha (αMEM) additionné de Glu, Asn, pénicilline et streptomycine ("αMEM supplémenté"), d'une part, et SP, BSA, 2ME, CaCl₂ et PB, d'autre part ;
- des substances à tester, à savoir Ex 1 et 5FU, 5FU étant une drogue cytotoxique détruisant toutes les cellules (normales ou pathologiques) entrées dans le cycle de la division cellulaire.

Les méthodes suivantes ont été utilisées. Le produit de l'exemple 1 a été injecté à la dose de 5 µg/injection sous un volume de 0,2 ml, et le 5FU à la dose de 150 mg/kg sous un volume de 0,2 ml. Les souris ont été réparties en lots de 5 animaux chacun, le lot témoin recevant une solution placebo composé de PBS.

### Premier protocole

Un lot témoin recoit le placebo et un lot reçoit Ex1 (5 µg/inj) une première fois par voie sous-cutanée, le matin à l'instant T=0. Ces lots recoivent le 5FU (150 mg/kg) par voie intrapéritonéale et le placebo ou Ex.1 (5 µg/inj) par voie sous-cutanée à l'instant T=+6 h. A l'instant T=+198 h (i.e. 8 jours après l'injection de 5FU) les souris sont sacrifiées.

### Deuxième protocole

Deux fois par jour, à 6 h d'intervalles, un lot témoin reçoit le placebo et un lot reçoit Exl par voie intrapéritonéale. 20 heures après, chaque lot reçoit 150 mg/kg de 5FU par voie intrapéritonéale sous un volume de 0,2 ml. Le jour de l'injection du 5FU est noté J=0 par commodité. A l'instant J=+5 un prélèvement sanguin au niveau du sinus orbital est réalisé. A l'instant J=+8, les souris sont sacrifiées.

### Analyse des mégacaryocytes et de leurs progéniteurs

Les souris sont sacrifiées par élongation cervicale. Les cellules de la moëlle osseuse sont récupérées à partir des 2 fémurs. 5.10⁴ cellules sont ensemencées dans des boîtes de Pétri, dans 1 ml de milieu alpha-MEM supplémenté contenant 10 % p/v de BSA, 10 % p/v de SP, 10 % p/v de 2ME, 10 % p/v de PB et 0,34 mg/ml de CaCl₂. Après formation du caillot, 3 boîtes de Pétri sont séchées avec du papier filtre puis fixées avec du paraformaldéhyde à 1 % p/v. On évalue (pour le témoin) le nombre des MK isolés après coloration à partir de ces boîtes. Les cellules contenues dans les autres boîtes sont cultivées pendant 7 ou 8 jours à 37°C en atmosphère humide contenant 5 % p/v de CO₂ . Comme précédemment les boîtes sont séchées avec du papier filtre puis fixées avec du paraformaldéhyde à 1 % p/v, les boîtes sont ensuite rincées 3 fois pendant 5 minutes avec du tampon phosphate à pH6. Pendant ces 7 ou 8 jours de culture, les colonies mégacaryocytaires ont pu se développer (une colonie est constituée de 3 MK ou plus) et le nombre des CFU-MK est évalué après coloration en s'appuyant sur la mise en évidence de l'activité acétylcholinestérase des MK murins. Les HPP-CFC forment des colonies visibles à l'oeil nu, leur diamètre étant supérieur ou égal à 0,5 mm. Le nombre de ces colonies est également déterminé par la coloration spécifique des MK suivie d'une coloration à l'hématoxyline. On dépose 3 ml de la solution permettant de réaliser la coloration des MK dans chaque boîte, on incube pendant 3-4 h à RT puis on rince à l'eau distillée.

### Analyse des cellules sanguines périphériques

Le sang est récupéré au niveau du sinus orbital à l'aide d'une pipette. Le jour où les souris sont sacrifiées, le sang est récupéré par ponction cardiaque. On détermine le nombre des plaquettes, des leucocytes ainsi que le taux d'hémoglobine par comptage hématopoïétique automatique au moyen d'un appareil approprié (Coulter T 890).

Les résultats obtenus selon le premier protocole et respectivement le deuxième protocole sont consignés dans le tableau II et respectivement le tableau III ci-après. Ces résultats mettent en évidence les propriétés cytoprotectrices du dodécapeptide de formule la selon l'invention.

Les expérimentations analogues entreprises avec le pF4 hautement purifié (dépourvu de TGFβ, fibrinogène, fibronectine, thrombospondine et vWF), le pF4 natif purifié et le pF4 recombinant montrent également l'utilité de ces produits en tant qu'agents cytoprotecteurs.

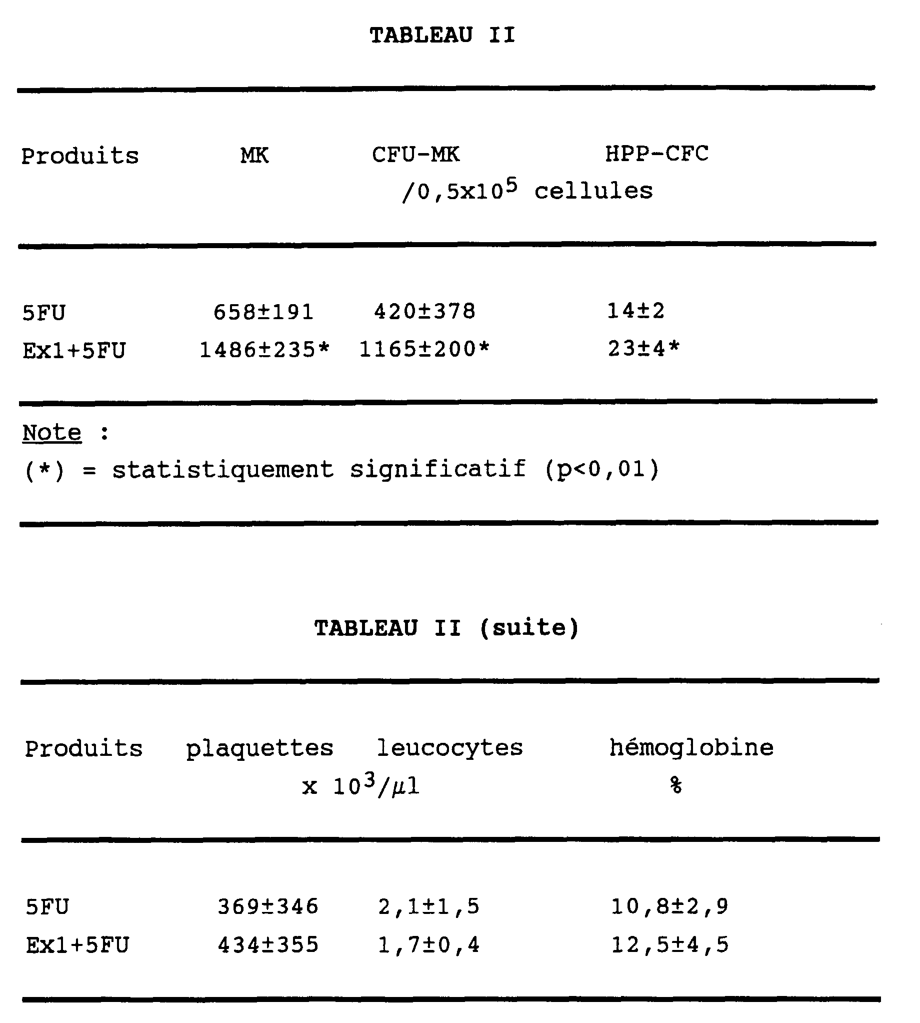

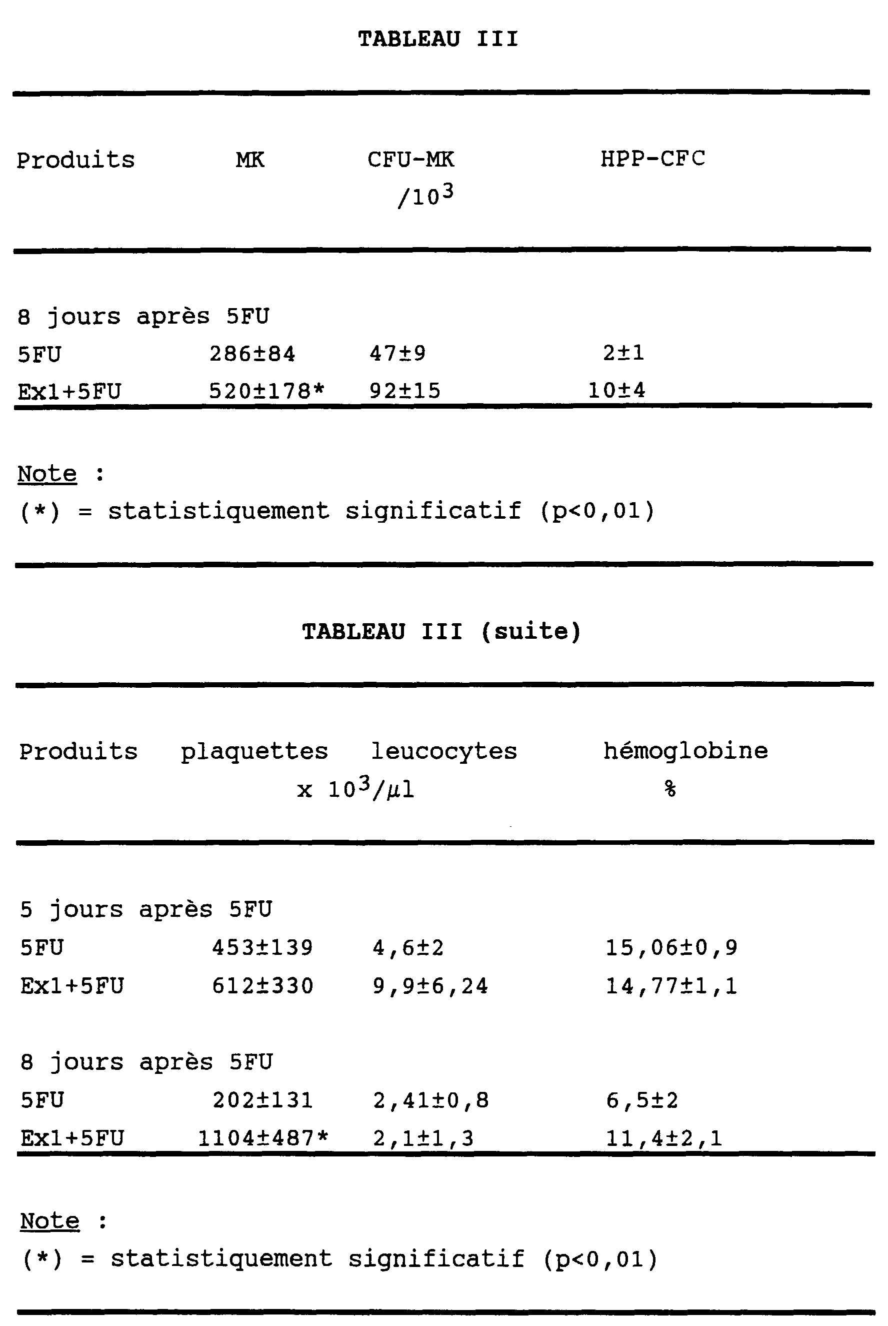

La liste des séquences (SEQ ID NO 1 à 14) est donnée ci-après. Par commodité dans cette liste, la séquence dite SEQ ID NO 3 a été représentée comme ayant 22 aminoacides, alors qu'en fait cette séquence comporte de 9 à 22 aminoacides.

### LISTE DE SEQUENCES

(1) INFORMATION GENERALE:
   (i) DEPOSANT:
      (A) NOM: Cabinet Fedit-Loriot (mandataire)
      (B) RUE: 38, avenue Hoche
      (C) VILLE: Paris
      (E) PAYS: France
      (F) CODE POSTAL: 75008
      (G) TELEPHONE: 1-42564235
      (H) TELECOPIE: 1-42898240
      (I) TELEX: 642587 F

      (A) NOM: Serbio
      (B) RUE: 125, avenue Louis Roche
      (C) VILLE: Gennevilliers
      (E) PAYS: France
      (F) CODE POSTAL: 92230

      (A) NOM: Institut des Vaisseaux et du Sang
      (B) RUE: 8, rue Guy Patin
      (C) VILLE: Paris
      (E) PAYS: France
      (F) CODE POSTAL: 75010
   (ii) TITRE DE L' INVENTION: "PEPTIDES MONOMERES ET DIMERES, UTILISATION EN TANT QU'AGENTS CYTOPROTECTEURS ET PROCEDE DE PREPARATION"
   (iii) NOMBRE DE SEQUENCES: 14
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: FR 9205990
      (B) DATE DE DEPOT: 18-MAI-1992
(2) INFORMATION POUR LA SEQ ID NO: 1:
   (i) CARACTERISTIQUES DE LA SEQUENCE:
      (A) LONGUEUR: 4 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 1
      (D) AUTRES RENSEIGNEMENTS: /note= ""Xaa signifie Leu, Ile, Nle, Val, Nva, Ahe, Acp ou cycloleucine""
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Xaa signifie Leu, Ile, Nle, Val, Nva, Ahe, Acp - ou cycloleucine""
   (xi) DESCRIPTION DE LA SEQUENCE: SEQ ID NO: 1:
   Xaa Cys Xaa Asx
   1
(2) INFORMATION POUR LA SEQ ID NO: 2:
   (i) CARACTERISTIQUES DE LA SEQUENCE:
      (A) LONGUEUR: 8 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 2
      (D) AUTRES RENSEIGNEMENTS: /note= ""Xaa signifie Gly, MeGly, EtGly ou Aib""
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Xaa Arg, Dbu, His, Lys ou Orn""
   (ix) CARACTERISTIQUE ADDITIONELLE:
      (A) NOM/CLE: Site modifié
      (B) EMPLACEMENT: 4
      (D) AUTRES RENSEIGNEMENTS: /note= ""Xaa signifie un aminoacide ayant un groupe latéral basique, acide ou amide""
   (ix) CARACTERISTIQUE ADDITIONELLE:
      (A) NOM/CLE: Site modifié
      (B) EMPLACEMENT: 5
      (D) AUTRES RENSEIGNEMENTS: /note= ""Xaa signifie Leu, Ile, Nle, Val, Nva, Ahe, Acp ou cycloleucine""
   (ix) CARACTERISTIQUE ADDITIONELLE:
      (A) NOM/CLE: Site modifié
      (B) EMPLACEMENT: 7
      (D) AUTRES RENSEIGNEMENTS: /note= ""Xaa signifie Leu, Ile, Nle, Val, Nva, Ahe, Acp ou cycloleucine""
   (xi) DESCRIPTION DE LA SEQUENCE: SEQ ID NO: 2:
   Asx Xaa Xaa Xaa Xaa Cys Xaa Asx
   1               5
(2) INFORMATION POUR LA SEQ ID NO: 3:
   (i) CARACTERISTIQUES DE LA SEQUENCE:
      (A) LONGUEUR: 22 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 1
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Peptide
      (B) EMPLACEMENT: 2
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 4
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 5
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 6
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 7
      (D) AUTRES RENSEIGNEMENTS: /note= ""Xaa signifie une simple liaison ou un aminoacide différent de cys""
   (ix) CARACTERISTIQUE ADDITIONELLE:
      (A) NOM/CLE: Site modifié
      (B) EMPLACEMENT: 8
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 9
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 10
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 12
      (D) AUTRES RENSEIGNEMENTS: /note= ""Xaa signifie Gly, MeGly, EtGly ou Aib""
   (ix) CARACTERISTIQUE ADDITIONELLE:
      (A) NOM/CLE: Site modifié
      (B) EMPLACEMENT: 13
      (D) AUTRES RENSEIGNEMENTS: /note= ""Xaa signifie Arg, Dbu,His, Lys ou Orn""
   (ix) CARACTERISTIQUE ADDITIONELLE:
      (A) NOM/CLE: Site modifié
      (B) EMPLACEMENT: 14
      (D) AUTRES RENSEIGNEMENTS: /note= ""Xaa est un aminoacide ayant un groupe latéral basique, acide ou amide""
   (ix) CARACTERISTIQUE ADDITIONELLE:
      (A) NOM/CLE: Site modifié
      (B) EMPLACEMENT: 15
      (D) AUTRES RENSEIGNEMENTS: /note= ""Xaa signifie Leu, Ile, Nle, Val, Nva, Ahe, Acp ou cycloleucine""
   (ix) CARACTERISTIQUE ADDITIONELLE:
      (A) NOM/CLE: Site modifié
      (B) EMPLACEMENT: 17
      (D) AUTRES RENSEIGNEMENTS: /note= ""Xaa signifie Leu, Ile, NLe, Val, Nva, Ahe, Acp ou cycloleucine""
   (ix) CARACTERISTIQUE ADDITIONELLE:
      (A) NOM/CLE: Site modifié
      (B) EMPLACEMENT: 19
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 20
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 21
      (D) AUTRES RENSEIGNEMENTS: /note= ""Xaa signifie une simple liaison ou un aminoacide différent de Cys""
   (ix) CARACTERISTIQUE ADDITIONELLE:
      (A) NOM/CLE: Site modifié
      (B) EMPLACEMENT: 22
      (D) AUTRES RENSEIGNEMENTS: /note= ""Xaa est un aminoacide cyclique""
   (xi) DESCRIPTION DE LA SEQUENCE: SEQ ID NO: 3:
   Xaa Xaa Xaa Xaa Xaa Xaa Xaa Xaa Xaa Xaa Asx Xaa Xaa Xaa Xaa Cys
   1               5                   10                  15
   Xaa Asx Xaa Xaa Xaa Xaa
               20
(2) INFORMATION POUR LA SEQ ID NO: 4:
   (i) CARACTERISTIQUES DE LA SEQUENCE:
      (A) LONGUEUR: 12 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 4:
   Asn Gly Arg Lys Ile Cys Leu Asp Leu Glu Ala Pro
   1               5                   10
(2) INFORMATION POUR LA SEQ ID NO: 5:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 4
      (D) AUTRES RENSEIGNEMENTS: /note= ""Xaa signifie cyclohexylalanine""
   (ix) CARACTERISTIQUE ADDITIONELLE:
      (A) NOM/CLE: Site modifié
      (B) EMPLACEMENT: 6
      (D) AUTRES RENSEIGNEMENTS: /note= ""Xaa signifie Orn""
   (ix) CARACTERISTIQUE ADDITIONELLE:
      (A) NOM/CLE: Site modifié
      (B) EMPLACEMENT: 8
      (D) AUTRES RENSEIGNEMENTS: /note= ""Xaa signifie Aib""
   (ix) CARACTERISTIQUE ADDITIONELLE:
      (A) NOM/CLE: Site modifié
      (B) EMPLACEMENT: 13
      (D) AUTRES RENSEIGNEMENTS: /note= ""Leu signifie Nle""
   (ix) CARACTERISTIQUE ADDITIONELLE:
      (A) NOM/CLE: Site modifié
      (B) EMPLACEMENT: 15
      (D) AUTRES RENSEIGNEMENTS: /note= ""Leu signifie Nle""
   (ix) CARACTERISTIQUE ADDITIONELLE:
      (A) NOM/CLE: Site modifié
      (B) EMPLACEMENT: 17
      (D) AUTRES RENSEIGNEMENTS: /note= ""Xaa signifie cyclohexylglycine""
   (ix) CARACTERISTIQUE ADDITIONELLE:
      (A) NOM/CLE: Site modifié
      (B) EMPLACEMENT: 18
      (D) AUTRES RENSEIGNEMENTS: /note= ""Xaa signifie 3Hyp ou 4Hyp""
   (xi) DESCRIPTION DE LA SEQUENCE: SEQ ID NO: 5:
   Gln Ile Ile Xaa Thr Xaa Asn Xaa Arg Lys Ile Cys Leu Asp Leu Gln
   1               5                   10                  15
   Xaa Xaa 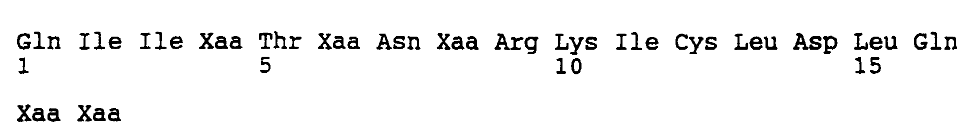
(2) INFORMATION POUR LA SEQ ID NO: 6:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 2
      (D) AUTRES RENSEIGNEMENTS: /note= ""Xaa signifie cycloleucine""
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Xaa signifie acide 2-azétidinecarboxylique""
   (ix) CARACTERISTIQUE ADDITIONELLE:
      (A) NOM/CLE: Site modifié
      (B) EMPLACEMENT: 4
      (D) AUTRES RENSEIGNEMENTS: /note= ""Xaa signifie (4-hydroxycyclohexyl)alanine""
   (ix) CARACTERISTIQUE ADDITIONELLE:
      (A) NOM/CLE: Site modifié
      (B) EMPLACEMENT: 15
      (D) AUTRES RENSEIGNEMENTS: /note= ""Leu signifie Nie""
   (ix) CARACTERISTIQUE ADDITIONELLE:
      (A) NOM/CLE: Site modifié
      (B) EMPLACEMENT: 18
      (D) AUTRES RENSEIGNEMENTS: /note= ""Xaa signifie acide 2-azétidinecarboxylique""
   (xi) DESCRIPTION DE LA SEQUENCE: SEQ ID NO: 6:
   Glu Xaa Xaa Xaa Ser His Asn Gly Arg Lys Ile Cys Leu Asp Leu Glu
   1               5                   10                  15
   Ala Xaa
(2) INFORMATION POUR LA SEQ ID NO: 7:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 7:
   Asp Gly Arg Lys Ile Cys Leu Asp Pro
   1               5
(2) INFORMATION POUR LA SEQ ID NO: 8:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 8:
   Asp Gly Arg Lys Leu Cys Leu Asp Pro
   1               5
(2) INFORMATION POUR LA SEQ ID NO: 9:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 9:
   Asp Gly Arg Glu Ile Cys Leu Asp Pro
   1               5
(2) INFORMATION POUR LA SEQ ID NO: 10:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 5
      (D) AUTRES RENSEIGNEMENTS: /note= ""Val signifie Nva""
   (xi) DESCRIPTION DE LA SEQUENCE: SEQ ID NO: 10:
   Asp Gly Arg Lys Val Cys Val Asp Pro
   1               5
(2) INFORMATION POUR LA SEQ ID NO: 11:
   (i) CARACTERISTIQUES DE LA SEQUENCE:
      (A) LONGUEUR: 10 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 2
      (D) AUTRES RENSEIGNEMENTS: /note= ""Xaa signifie Aib""
   (ix) CARACTERISTIQUE ADDITIONELLE:
      (A) NOM/CLE: Site modifié
      (B) EMPLACEMENT: 4
      (D) AUTRES RENSEIGNEMENTS: /note= ""Xaa signifie Dbu""
   (xi) DESCRIPTION DE LA SEQUENCE: SEQ ID NO: 11:
   Asn Xaa Arg Xaa Ile Cys Leu Asp Leu Pro
   1               5                   10
(2) INFORMATION POUR LA SEQ ID NO: 12:
   (i) CARACTERISTIQUES DE LA SEQUENCE:
      (A) LONGUEUR: 16 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Xaa signifie Dbu""
   (ix) CARACTERISTIQUE ADDITIONELLE:
      (A) NOM/CLE: Site modifié
      (B) EMPLACEMENT: 15
      (D) AUTRES RENSEIGNEMENTS: /note= ""Xaa signifie Aad""
   (xi) DESCRIPTION DE LA SEQUENCE: SEQ ID NO: 12:
   Ile Val Xaa Thr Asp Gly Arg Lys Ile Cys Phe Asp Pro Val Xaa Pro
   1               5                   10                  15
(2) INFORMATION POUR LA SEQ ID NO: 13:
   (i) CARACTERISTIQUES DE LA SEQUENCE:
      (A) LONGUEUR: 12 acides aminés
      (B) TYPE: acide aminé
      (D) CONFIGURATION: linéaire
   (ii) TYPE DE MOLECULE: peptide
   (ix) CARACTERISTIQUE ADDITIONELLE:
      (A) NOM/CLE: Site modifié
      (B) EMPLACEMENT: 1
      (D) AUTRES RENSEIGNEMENTS: /note= ""Val signifie Nva""
   (ix) CARACTERISTIQUE ADDITIONELLE:
      (A) NOM/CLE: Site modifié
      (B) EMPLACEMENT: 2
      (D) AUTRES RENSEIGNEMENTS: /note= ""Xaa signifie Dpr""
   (ix) CARACTERISTIQUE ADDITIONELLE:
      (A) NOM/CLE: Site modifié
      (B) EMPLACEMENT: 3
      (D) AUTRES RENSEIGNEMENTS: /note= ""Val signifie Nva""
   (ix) CARACTERISTIQUE ADDITIONELLE:
      (A) NOM/CLE: Site modifié
      (B) EMPLACEMENT: 10
      (D) AUTRES RENSEIGNEMENTS: /note= ""Xaa signifie Ahe""
   (xi) DESCRIPTION DE LA SEQUENCE: SEQ ID NO: 13:
   Val Xaa Val Asp Gly His Lys Ile Cys Xaa Asp Pro
   1               5                   10
(2) INFORMATION POUR LA SEQ ID NO: 14:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: peptide
   (xi) DESCRIPTION DE LA SEQUENCE: SEQ ID NO: 14:
   Val Ile Val Lys Leu Ser Asp Gly Arg Glu Leu Cys Leu Asp Pro Lys
   1               5                   10                  15
   Glu Asn

## Revendications

1. Utilisation d'une substance peptidique, ladite utilisation étant caractérisée en ce que l'on fait appel à une substance peptidique comportant dans sa structure
(a) un premier fragment de formule :
Asx-A₁-A₂ (F₁)
dans laquelle,
Asx représente Asp ou Asn,
A₁ représente Gly, MeGly, EtGly ou Aib,
A₂ représente Arg, Dbu, His, Lys ou Orn, et
(b) un second fragment de formule :
A₃-Cys-A₄-Asx (F₂)
dans laquelle,
A₃ représente Leu, Ile, Nle, Val, Nva, Ahe, Acp ou Cle,
A₄ représente Leu, Ile, Nle, Val, Nva, Ahe, Acp ou Cle, et
Asx représente Asp ou Asn comme indiqué ci-dessus, le second fragment (F₂) étant plus proche de l'extrémité C-terminale que le premier fragment (F₁),
pour la préparation d'un médicament cytoprotecteur utile vis-à-vis des agressions cytotoxiques.

2. Utilisation suivant la revendication 1, caractérisée en ce que l'on fait appel à une substance peptidique qui
(1) a des propriétés antimégacaryocytaires, et
(2) comporte dans sa structure
(a) un premier fragment de formule :
Asx-A₁-A₂ (F₁)
dans laquelle
Asx représente Asp ou Asn,
A₁ représente Gly, MeGly, EtGly ou Aib,
A₂ représente Arg, Dbu, His, Lys ou Orn, et
(b) un second fragment de formule
A₃-Cys-A₄-Asx (F₂)
dans laquelle,
A₃ représente Leu, Ile, Nle, Val, Nva, Ahe, Acp ou Cle,
A₄ représente Leu, Ile, Nle, Val, Nva, Ahe, Acp ou Cle, et
Asx représente Asp ou Asn comme indiqué ci-dessus, le second fragment (F₂) étant plus proche de l'extrémité C-terminale que le premier fragment (F₁),
pour la préparation d'un médicament cytoprotecteur vis-à-vis des thérapeutiques cytotoxiques, notamment les chimiothérapies et les autogreffes.

3. Utilisation suivant la revendication 1 ou 2, caractérisée en ce que la substance peptidique comporte le fragment (F₁) relié au fragment (F₂) par l'intermédiaire d'un aminoacide, selon la structure
F₁-A₅-F₂ (F₃)
dans laquelle A₅ est un reste aminoacide comportant un groupe latéral basique, acide ou amide, et F₁ et F₂ sont définis comme indiqué ci-dessus.

4. Utilisation suivant la revendication 3, caractérisée en ce que A₅ est Arg, Lys, His, Orn, Dbu, Glu, Gln, Asp, Asn, Aad, Dpr.

5. Utilisation suivant l'une quelconque des revendications 1 à 3, caractérisée en ce que la substance peptidique a une structure monomère de formule :
R-B₁-Asx-A₁-A₂-A₅-A₃-Cys-A₄-Asx-B₂-R' (I)
ou une structure dimère de formule : 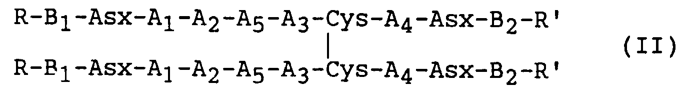 dans lesquelles,
A₁, A₂, A₃, A₄, et A₅ sont définis comme indiqué ci-dessus,
B₁ représente une simple liaison, un groupe aminoacide ou un reste peptidique ayant de 2 à 10 groupes aminoacides,
B₂ représente un groupe aminoacide ou un reste peptidique ayant de 2 à 14 groupes aminoacide, l'aminoacide de l'extrémité C-terminale de B₂ ayant une structure cyclique et étant choisi parmi l'ensemble constitué par Pro, 3Hyp, 4Hyp, ATC, Aze, Pip, Cle, ACC et Pyr,
R est H ou un groupe protecteur de la fonction NH₂ de l'extrémité N-terminale, et
R' est OH ou un groupe protecteur de la fonction COOH de l'extrémité C-terminale.

6. Utilisation suivant la revendication 5, caractérisée en ce que (i) le ou les aminoacides inclus dans B₁ et (ii) le ou les aminoacides inclus dans B₂ et autres que celui de l'extrémité C-terminale, sont choisis parmi l'ensemble constitué par Ala, Phe, cyclohexylalanine (CHA), cyclohexylglycine (CHG), cyclopentylalanine (CPA), cyclopentylglycine (CPG), Tyr, 3-(4-hydroxycyclohexyl)glycine (CHU), 3-(4-hydroxycyclohexyl)alanine (CHT), Leu, Ile, Nle, Val, Nva, Ahe, Acp, Arg, Lys, His, Orn, Dbu, Glu, Gln, Asp, Asn, Aad, Dpr, Ser, Gly, MeGly, EtGly, Aib, Thr, acide pipécolique (Pip), Pro, 3Hyp, 4Hyp, ATC, Aze, Pip, Cle, ACC et Pyr.

7. Utilisation suivant l'une quelconque des revendications 1, 2 et 3, caractérisée en ce que la substance peptidique est choisie parmi l'ensemble constitué par le pF4, le pF4 natif, le pF4 recombinant et leurs mélanges.

8. Utilisation suivant l'une quelconque des revendications 1, 2 et 3, caractérisée en ce que la substance peptidique est choisie parmi l'ensemble constitué par le LA-pF4, la βTG, l'IL8, le CTAP-III et leurs mélanges.

9. Utilisation suivant la revendication 5, caractérisée en ce que la substance peptidique est choisie parmi l'ensemble constitué par le dodécapeptide monomère de formule : son dimère de formule : et leurs mélanges.

10. Substance peptidique caractérisée en ce qu'elle a une structure monomère de formule :
R-B₁-Asx-A₁-A₂-A₅-A₃-Cys-A₄-Asx-B₂-R' (I)
ou une structure dimère de formule : 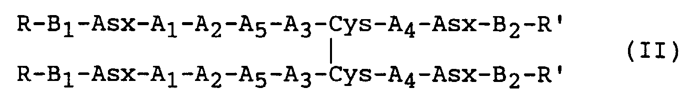 où A₁, A₂, A₃, A₄ et A₅ sont définis comme indiqué ci-dessus,
B₁ représente une simple liaison, un groupe aminoacide ou un reste peptidique ayant de 2 à 10 groupes aminoacides,
B₂ représente un groupe aminoacide ou un reste peptidique ayant de 2 à 14 groupes aminoacide, l'aminoacide de l'extrémité C-terminale de B₂ ayant une structure cyclique et étant choisi parmi l'ensemble constitué par Pro, 3Hyp, 4Hyp, ATC, Aze, Pip, Cle, ACC et Pyr,
R est H ou un groupe protecteur de la fonction NH₂ de l'extrémité N-terminale, et
R' est OH ou un groupe protecteur de la fonction COOH de l'extrémité C-terminale.

11. Substance peptidique suivant la revendication 10, caractérisée en ce que (i) le ou les aminoacides inclus dans B₁ et (ii) le ou les aminoacides inclus dans B₂ et autres que celui de l'extrémité C-terminale, sont choisis parmi l'ensemble constitué par Ala, Phe, cyclohexylalanine (CHA), cyclohexylglycine (CHG), cyclopentylalanine (CPA), cyclopentylglycine (CPG), Tyr, (4-hydroxycyclohexyl)glycine (CHU), (4-hydroxycyclohexyl)alanine (CHT), Leu, Ile, Nle, Val, Nva, Ahe, Acp, Arg, Lys, His, Orn, Dbu, Glu, Gln, Asp, Asn, Aad, Dpr, Ser, Gly, MeGly, EtGly, Aib, Thr, acide pipécolique (Pip), Pro, 3Hyp, 4Hyp, ATC, Aze, Pip, Cle, ACC et Pyr.

12. Substance peptidique suivant la revendication 10, caractérisée en ce qu'elle est choisie parmi l'ensemble constitué par le dodécapeptide monomère de formule : son dimère de formule : et leurs mélanges.

13. Procédé de préparation d'un composé de formule I ou II, ledit procédé, qui met en oeuvre une synthèse peptidique séquentielle dans un milieu réactionnel et hétérogène et une étape de purification, étant caractérisé en ce qu'il comprend
(a) l'utilisation d'un agent réducteur
(i) dans le milieu réactionnel à partir de l'instant où Cys intervient dans la synthèse dudit peptide monomère, ou ultérieurement,
(ii) avant et/ou après purification,
afin de réduire substantiellement ou d'empêcher la production du peptide dimère de formule II qui résulte de la formation d'un pont disulfure -S-S- entre les Cys de deux chaînes monomères, pour l'obtention d'un composé de formule I, et
(b) si nécessaire, la formation dudit pont -S-S- par l'intermédiaire des Cys de deux chaînes monomères, pour l'obtention d'un composé de formule II.

14. Utilisation d'une substance peptidique choisie parmi l'ensemble constitué par les peptides monomères de formule I les peptides dimères de formule II selon la revendication 10 et leurs mélanges, pour la préparation d'un médicament antimégacaryocytaire vis-à-vis des thrombocytémies.

15. Utilisation suivant la revendication 14, dans laquelle la substance peptidique est choisie parmi le dodécapeptide monomère de formule : son dimère de formule : et leurs mélanges.

## Claims

1. Use of a peptide substance, wherein a peptide substance whose structure contains
(a) a first fragment of the formula
Asx-A₁-A₂ (F₁)
in which
Asx is Asp or Asn,
A₁ is Gly, MeGly, EtGly or Aib and
A₂ is Arg, Dbu, His, Lys or Orn, and
(b) a second fragment of the formula
A₃-Cys-A₄-Asx (F₂)
in which
A₃ is Leu, Ile, Nle, Val, Nva, Ahe, Acp or Cle,
A₄ is Leu, Ile, Nle, Val, Nva, Ahe, Acp or Cle and
Asx is Asp or Asn, as indicated above, the second fragment (F₂) being nearer the C-terminal end than the first fragment (F₁),
is used for the preparation of a cytoprotective drug useful for combating cytotoxic aggression.

2. Use according to claim 1 wherein a peptide substance
(1) which has megakaryocyte-inhibiting properties and
(2) whose structure contains
(a) a first fragment of the formula
Asx-A₁-A₂ (F₁)
in which
Asx is Asp or Asn,
A₁ is Gly, MeGly, EtGly or Aib and
A₂ is Arg, Dbu, His, Lys or Orn, and
(b) a second fragment of the formula
A₃-Cys-A₄-Asx (F₂)
in which
A₂ is Leu, Ile, Nle, Val, Nva, Ahe, Acp or Cle,
A₄ is Leu, Ile, Nle, Val, Nva, Ahe, Acp or Cle and
Asx is Asp or Asn, as indicated above, the second fragment (F₂) being nearer the C-terminal end than the first fragment (F₁),
is used for the preparation of a cytoprotective drug for combating cytotoxic therapy, especially- chemotherapy and autografts.

3. Use according to claim 1 or 2 wherein the peptide substance contains the fragment (F₁) joined to the fragment (F₂) *via* an amino acid to give the structure
F₁-A₅-F₂ (F₃)
in which A₅ is an amino acid residue containing a basic, acidic or amido side-group and F₁ and F₂ are defined as indicated above.

4. Use according to claim 3 wherein A₅ is Arg, Lys, His, Orn, Dbu, Glu, Gln, Asp, Asn, Aad or Dpr.

5. Use according to any one of claims 1 to 3 wherein the peptide substance has a monomeric structure of the formula
R-B₁-Asx-A₁-A₂-A₅-A₃-Cys-A₄-Asx-B₂-R' (I)
or a dimeric structure of the formula 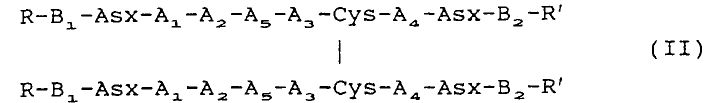 in which A₁, A₂, A₃, A₄ and A₅ are defined as indicated above,
B₁ is a single bond, an amino acid group or a peptide residue having from 2 to 10 amino acid groups,
B₂ is an amino acid group or a peptide residue having from 2 to 14 amino acid groups, the amino acid at the C-terminal end of B₂ having a cyclic structure and being selected from the group consisting of Pro, 3Hyp, 4Hyp, ATC, Aze, Pip, Cle, ACC and Pyr,
R is H or a protecting group for the NH₂ group at the N-terminal end, and
R' is OH or a protecting group for the COOH group at the C-terminal end.

6. Use according to claim 5 wherein (i) the amino acid or amino acids included in B₁ and (ii) the amino acid or amino acids included in B₂, other than the one at the C-terminal end, are selected from the group consisting of Ala, Phe, cyclohexylalanine (CHA), cyclohexylglycine (CHG), cyclopentylalanine (CPA), cyclopentylglycine (CPG), Tyr, 3-(4-hydroxycyclohexyl)glycine (CHU), 3-(4-hydroxycyclohexyl)alanine (CHT), Leu, Ile, Nle, Val, Nva, Ahe, Acp, Arg, Lys, His, Orn, Dbu, Glu, GIn, Asp, Asn, Aad, Dpr, Ser, Gly, MeGly, EtGly, Aib, Thr, pipecolic acid (Pip), Pro, 3Hyp, 4Hyp, ATC, Aze, Pip, Cle, ACC and Pyr.

7. Use according to any one of claims 1, 2 and 3 wherein the peptide substance is selected from the group consisting of pF4, native pF4, recombinant pF4 and mixtures thereof.

8. Use according to any one of claims 1, 2 and 3 wherein the peptide substance is selected from the group consisting of LA-pF4, βTG, IL8, CTAP-III and mixtures thereof.

9. Use according to claim 5 wherein the peptide substance is selected from the group consisting of the monomeric dodecapeptide of the formula its dimer of the formula and mixtures thereof.

10. A peptide substance which has a monomeric structure of the formula
R-B₁-Asx-A₁-A₂-A₅-A₃-Cys-A₄-Asx-B₂-R' (I)
or a dimeric structure of the formula 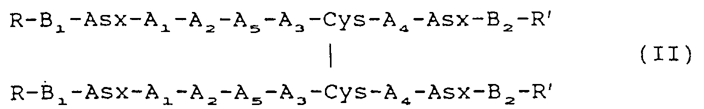 in which A₁, A₂, A₃, A₄ and A₅ are defined as indicated above,
B₁ is a single bond, an amino acid group or a peptide residue having from 2 to 10 amino acid groups,
B₂ is an amino acid group or a peptide residue having from 2 to 14 amino acid groups, the amino acid at the C-terminal end of B₂ having a cyclic structure and being selected from the group consisting of Pro, 3Hyp, 4Hyp, ATC, Aze, Pip, Cle, ACC and Pyr,
R is H or a protecting group for the NH₂ group at the N-terminal end, and
R' is OH or a protecting group for the COOH group at the C-terminal end.

11. A peptide substance according to claim 10 wherein (i) the amino acid or amino acids included in B₁ and (ii) the amino acid or amino acids included in B₂, other than the one at the C-terminal end, are selected from the group consisting of Ala, Phe, cyclohexylalanine (CHA), cyclohexylglycine (CHG), cyclopentylalanine (CPA), cyclopentylglycine (CPG), Tyr, (4-hydroxycyclohexyl)glycine (CHU), (4-hydroxycyclohexyl)alanine (CHT), Leu, Ile, Nle, Val, Nva, Ahe, Acp, Arg, Lys, His, Orn, Dbu, Glu, Gln, Asp, Asn, Aad, Dpr, Ser, Gly, MeGly, EtGly, Aib, Thr, pipecolic acid (Pip), Pro, 3Hyp, 4Hyp, ATC, Aze, Pip, Cle, ACC and Pyr.

12. A peptide substance according to claim 10 which is selected from the group consisting of the monomeric dodecapeptide of the formula its dimer of the formula and mixtures thereof.

13. A method of preparing a compound of formula I or II, said method, which involves a sequential peptide synthesis in a heterogeneous reaction medium and a purification step, comprising
(a) using a reducing agent
(i) in the reaction medium as from the moment when Cys enters the synthesis of said monomeric peptide, or subsequently,
(ii) before and/or after purification,
so as to reduce substantially or prevent the production of the dimeric peptide of formula II which results from the formation of a disulfide bridge -S-S- between the Cys of two monomeric chains, in order to obtain a compound of formula I, and
(b) if necessary, forming said -S-S- bridge *via* the Cys of two monomeric chains, in order to obtain a compound of formula II.

14. Use of a peptide substance selected from the group consisting of the monomeric peptides of formula I and the dimeric peptides of formula II according to claim 10 and mixtures thereof for the preparation of a megakaryocyte-inhibiting drug for combating thrombocytemia.

15. Use according to claim 14 wherein the peptide substance is selected from the monomeric dodecapeptide of the formula its dimer of the formula and mixtures thereof.

## Patentansprüche

1. Verwendung einer Peptidisubstanz zur Herstellung eines cytoprotektischen Medikaments gegen cytotoxische Aggressionen,
**dadurch gekennzeichnet**, **daß**
eine Peptidsubstanz mit der Struktur
(a) einem ersten Bruchstück der Formel:
Asx-A₁-A₂ (F_{1),}
in dem
Asx Asp oder Asn,
A₁ Gly, MeGly, EtGly oder Aib,
A₂ Arg, Dbu, His, Lys oder Orn sind und
(b) einem zweiten Bruchstück der Formel:
A₃-Cys-A₄-Asx (F₂),
in dem
A₃ Leu, Ile, Nle, Val, Nva, Ahe, Acp oder Cle,
A₄ Leu, Ile, Nle, Val, Nva, Ahe, Acp oder Cle sind, und
Asx Asp oder Asn wie vorstehend definiert sind, verwendet wird, wobei das zweite Bruchstück (F₂) näher am C-terminalen Ende als das erste Bruchstück (F₁) liegt.

2. Verwendung nach Anspruch 1,
**dadurch gekennzeichnet**, **daß**
eine Peptidsubstanz zur Herstellung eines cytoprotektischen Medikaments zu cytotoxischen Therapien, insbesondere Chemotherapien und Autotransplantaten
(1) mit antimegacaryocytären Eigenschaften und
(2) mit der Struktur
(a) einem ersten Bruchstück der Formel:
Asx-A₁-A₂ (F1),
in dem
Asx Asp oder Asn,
A₁ Gly, MeGly, EtGly oder Aib,
A₂ Arg, Dbu, His, Lys oder Orn sind und
(b) einem zweiten Bruchstück der Formel
A₃-Cys-A₄-Asx (F2),
in dem
A₃ Leu, Ile, Nle, Val, Nva, Ahe, Acp oder Cle,
A₄ Leu, Ile, Nle, Val, Nva, Ahe, Acp oder Cle sind, und
Asx Asp oder Asn wie vorstehend definiert sind, wobei das zweite Bruchstück (F₂) naher am C-terminalen Ende als das erste Bruchstück (F1) liegt, verwendet wird.

3. Verwendung nach Anspruch 1 oder 2,
**dadurch gekennzeichnet, daß**
das Bruchstück (F₁) mit dem Bruchstück (F₂) der Peptidsubstanz über eine Aminosäure gemäß der Struktur
F₁-A₅-F₂ (F₃),
verbunden ist, in der A₅ ein Aminosäurerest mit einer basischen, saueren oder amidischen Seitengruppe und F₁ und F₂ wie vorstehend definiert sind.

4. Verwendung nach Anspruch 3,
**dadurch gekennzeichnet, daß**
A₅ Arg, Lys, His, Orn, Dbu, Glu, Gln, Asp, Asn, Aad, Dpr sind.

5. Verwendung nach einem der Ansprüche 1 bis 3,
**dadurch gekennzeichnet, daß**
die Peptidsubstanz eine Monomerstruktur der Formel:
R-B₁-Asx-A₁-A₂-A₅-A₃-Cys-A₄-Asx-B₂-R' (I)
oder eine Dimerstruktur der Formel: 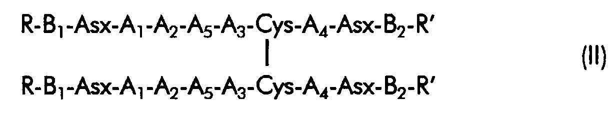 aufweist,
in dem
A₁, A₂, A₃, A₄ und A₅ wie vorstehend definiert sind,
B₁ eine Einfach bindung, eine Aminosäuregruppe oder ein Peptidrest mit 2 bis 10 Aminosäuregruppen, B₂ eine Aminosäuregruppe oder ein Peptidrest mit 2 bis 14 Aminosäuren, wobei die Aminosäure am C-terminalen Ende von B₂ eine cyklische Struktur aufweist und aus Pro, 3Hyp, 4Hyp, ATC, Aze, Pip, Cle, ACC und Pyr ausgewählt ist,
R H oder eine Schutzgruppe für die NH₂-Funktion des N-terminalen Endes und R' OH oder eine Schutzgruppe für die COOH-Funktion des C-terminalen Endes sind.

6. Verwendung nach Anspruch 5,
**dadurch gekennzeichnet, daß**
(i) die in B₁ enthaltene(n) Aminosäure(n) und (ii) in B₂ und andere(n) am C-terminalen Ende enthaltene(n) Aminosäure(n) aus Ala, Phe, Cyclohexylalanin (CHA), Cyclohexylglycin (CHG), Cyclopentylalanin (CPA), Cyclopentylglycin (CPG), Tyr, 3-(4-Hydroxycyclohexyl)glycin (CHU), 3-(4-Hydroxycyclohexyl)alanin (CHT), Leu, Ile, Nle, Val, Nva, Ahe, Acp, Arg, Lys, His, Orn, Dbu, Glu, Gln, Asp, Asn, Aad, Dpr, Ser, Gly, MeGly, EtGLy, Aib, Thr, Pipecolinsäure (Pip), Pro, 3Hyp, 4Hyp, ATC, Aze, Pip, Cle, ACC und Pyr ausgewählt sind.

7. Verwendung nach einem der Ansprüche 1, 2 und 3,
**dadurch gekennzeichnet, daß**
die Peptidsubstanz aus pF4, nativem pF4, rekombinantem pF4 und deren Mischungen ausgewählt ist.

8. Verwendung nach einem der Ansprüche 1, 2 und 3,
**dadurch gekennzeichnet, daß**
die Peptidsubstanz aus LA-pF4, βTG, IL8, CTAP-III und deren Mischungen ausgewählt ist.

9. Verwendung nach Anspruch 5,
**dadurch gekennzeichnet, daß**
die Peptidsubstanz aus einem Dodecapeptidmonomer der Formel: seinem Dimer der Formel: und deren Mischungen ausgewählt ist.

10. Peptidsubstanz
**gekennzeichnet durch**
eine Monomerstruktur der Formel:
R-B₁-Asx-A₁-A₂-A₅-A₃-Cys-A₄-Asx-B₂-R' (I)
oder eine Dimerstruktur der Formel: 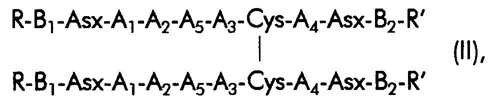 in der A₁, A₂, A₃, A₄ und A₅ wie vorstehend definiert sind,
B₁ eine Einfachbindung, eine Aminosäuregrupe oder ein Peptidrest mit 2 bis 10 Aminosäuregruppen,
B₂ eine Aminosäuregruppe oder ein Peptidrest mit 2 bis 14 Aminosäurengruppen, wobei die am C-terminalen Ende gelegene Aminosäure B₂ eine cyklische Struktur aufweist und aus Pro, 3Hyp, 4Hyp, ATC, Aze, Pip, Cle, ACC und Pyr ausgewählt ist,
R H oder eine Schutzgruppe für die NH₂-Funktion des N-terminalen Endes und
R' OH oder eine Schutzgruppe für die COOH-Funktion am C-terminalen Ende sind.

11. Peptidsubstanz nach Anspruch 10,
**dadurch gekennzeichnet, daß**
(i) die in B₁ enthaltene(n) Aminosäure(n) und (ii) in B₂ und anderen am C-terminalen Ende enthaltene(n) Aminosäure(n) aus Ala, Phe, Cyclohexylalanin (CHA), Cyclohexylglycin (CHG), Cyclopentylalanin (CPA), Cyclopentylglycin (CPG), Tyr, 3-(4-Hydroxycyclohexyl)glycin (CHU), 3-(4-Hydroxycyclohexyl)alanin (CHT), Leu, Ile, Nle, Val, Nva, Ahe, Acp, Arg, Lys, His, Orn, Dbu, Glu, Gln, Asp, Asn, Aad, Dpr, Ser, Gly, MeGly, EtGLy, Aib, Thr, Pipecolinsäure (Pip), Pro, 3Hyp, 4Hyp, ATC, Aze, Pip, Cle, ACC und Pyr ausgewählt sind.

12. Peptidsubstanz nach Anspruch 10,
**dadurch gekennzeichnet, daß**
die Peptidsubstanz aus einem Dodecapeptidmonomer der Formel: seinem Dimer der Formel: und deren Mischungen ausgewählt ist.

13. Verfahren zum Herstellen einer Zusammensetzung der Formel I oder II, wobei eine sequentielle Peptidsynthese in einem heterogenen Reaktionsmedium und eine Reinigungsphase durchgeführt wird,
**dadurch gekennzeichnet, daß**
es
(a) die Verwendung eines Reduktionsmittels
(i) in einem Reaktionsmedium beginnend mit dem Zeitpunkt, zu dem Cys auf die Synthese des Peptidmonomers einwirkt, oder später,
(ii) vor und/oder nach der Reinigung umfaßt,
um im wesentlichen die Produktion eines Peptiddimers der Formel II zu reduzieren oder zu verhindern, das zur Bildung einer S-S-Disulfid- Brücke zwischen zwischen den Cys der zwei monomeren Ketten führt, um eine Zusammensetzung der Formel I zu erhalten, und
(b) nötigenfalls die Bildung der S-S-Brücke mittels der Cys der beiden Monomerketten, um eine Zusammensetzung der Formel II zu erhalten.

14. Verwendung einer aus den Peptidmonomeren der Formel I und den Peptiddimeren der Formel II nach Anspruch 10 ausgewählten Peptidsubstanz und deren Mischungen zur Herstellung eines antimegacaryocytären Medikaments gegen die Thrombocytämie.

15. Verwendung nach Anspruch 14, wobei die Peptidsubstanz aus einem Dodecapeptidmonomer der Formel: ihrem Dimer der Formel: und deren Mischungen ausgewähltist.
